# EUROPEAN PATENT SPECIFICATION

(11) **EP 0 683 977 B1**
(45) Date of publication and mention of the grant of the patent: **02.08.2000**
(21) Application number: 95107083.8
(22) Date of filing: 10.05.1995
(51) Int. Cl.: A01M 1/20, A61L 9/12

(54) **Sustained release preparations**
Präparat mit verzörgerter Wirkstoffabgabe
Préparation du type à libération prolongée

(30) Priority: 24.05.1994 JP 10964394
(43) Date of publication of application: 29.11.1995
(73) Proprietor: Shin-Etsu Chemical Co., Ltd., Chiyoda-ku Tokyo 100 (JP)
(72) Inventor: Ogawa, Kinya, Shin-Etsu Chemical Co., Ltd., Tokyo (JP); Itoh, Kenichi, Shin-Etsu Chemical Co., Ltd., Nakakubiki-gun, Niigata 942-01 (JP); Suzuki, Hiroshi, Shin-Etsu Chemical Co., Ltd., Nakakubiki-gun, Niigata 942-01 (JP)
(74) Representative: Tiedtke, Harro, Dipl.-Ing.

(56) References cited:
- EP-A- 0 273 197
- WO-A-93/00115
- US-A- 3 698 974
- US-A- 4 834 745
- US-A- 5 071 704

## Description

The present invention relates to a sustained release preparation which permits gradual release of volatile ingredients such as pheromones, repellents, perfumes and insecticides and which is effective, in particular, as a sustained release dispenser for releasing pheromones to control occurrence of harmful insects.

There has been required for the development of a pharmaceutical or agricultural dispenser which permits gradual release of a volatile ingredient to ensure a long lasting effect thereof. The mating disruption method must satisfy this requirement, because this method which is utilized for control of agriculturally harmful insects through release of, for instance, a sex pheromone at a predetermined concentration over a long time period. The term "mating disruption method" herein means a method comprising, 1) making a sex pheromone drift in fields at a concentration substantially higher than that released from harmful insects per se, 2) to thus lower the communication ability of the harmful insects such as an ability of male or female insects to recognize the individual opposite sex and to confirm the positions thereof, 3) and to thereby disturb mating of the insects. There has been used a sustained release dispenser containing a pheromone as a physiologically active substance of a harmful insect as a communication-disturbing agent. Such sustained release dispenser may be in various forms, for instance, those packed in microcapsules as disclosed in U.S. Patent Nos. 2,800,457, 2,800,458 and 3,577,515; those supported by carriers as disclosed in U.S. Patent No. 4,160,335; those packed in tubes as disclosed in U.S. Patent Nos. 4,600,146 and 4,834,745; or those packed in bottle-like containers as disclosed in European Patent No. 273,197. The sustained release dispenser packed in a container having a large capacity, i.e., a reservoir type one is one of the leading mainstream thereof.

Containers for such reservoir type sustained release dispensers have been produced from a variety of plastics selected depending on the physical properties of individual volatile ingredients. When using such a reservoir type dispenser, a liquid and volatile ingredient absorbed in the plastic wall of the container can be vaporized from the outer surface thereof and diffuse into the air. Therefore, the release rate thereof is approximately proportional to the outer surface area of the reservoir which absorbs the liquid ingredient. The release rate has conventionally been controlled by adjusting the size of the outer surface area through appropriate selection of plastic materials for the reservoir and shapes thereof. However, the reservoir type sustained release dispenser suffers from a problem in that the amount of the liquid remaining in the reservoir decreases as the ingredient is released and this results in a decrease of the surface area wetted with the liquid ingredient through absorption thereof and hence the reduction in the amount of the drug released therefrom during the latter half of the ingredient-release, as clearly described in J. Economical Entomology, 78, No. 6, 1985.

US-A-5,071,704 and WO 93/00115 disclose a device for controlled release of volatile ingredients. The device is a multi-layered laminate consisting of a reservoir layer which incorporates a porous reservoir layer which incorporates an active compound in its pores, an impermeable membrane layer adjacent to the reservoir layer and a diffusion rate limiting membrane layer adjacent to the reservoir layer.

The present invention has been developed for solving the foregoing problems associated with the conventional techniques and accordingly, it is an object of the present invention to provide a sustained release dispenser which permits uniform release of a volatile ingredient over a long time period even if the amount of the liquid ingredient remaining in a container is reduced.

According to the present invention, the foregoing object can effectively be accomplished by providing a sustained release dispenser according to claim 1.
Fig. 1 is a cross-sectional perspective view illustrating main parts of an embodiment of the sustained release dispenser according to the present invention;
Fig. 2 is a cross-sectional perspective view illustrating main parts of another embodiment of the sustained release dispenser according to the present invention;
Fig. 3 is a diagram showing a change, with time, of the height of an HDDA liquid remaining in a tube;
Fig. 4 is a diagram showing a change, with time, of the amount of released HDDA, and
Fig. 5 is a diagram showing a change, with time, of the amount of released DDA.

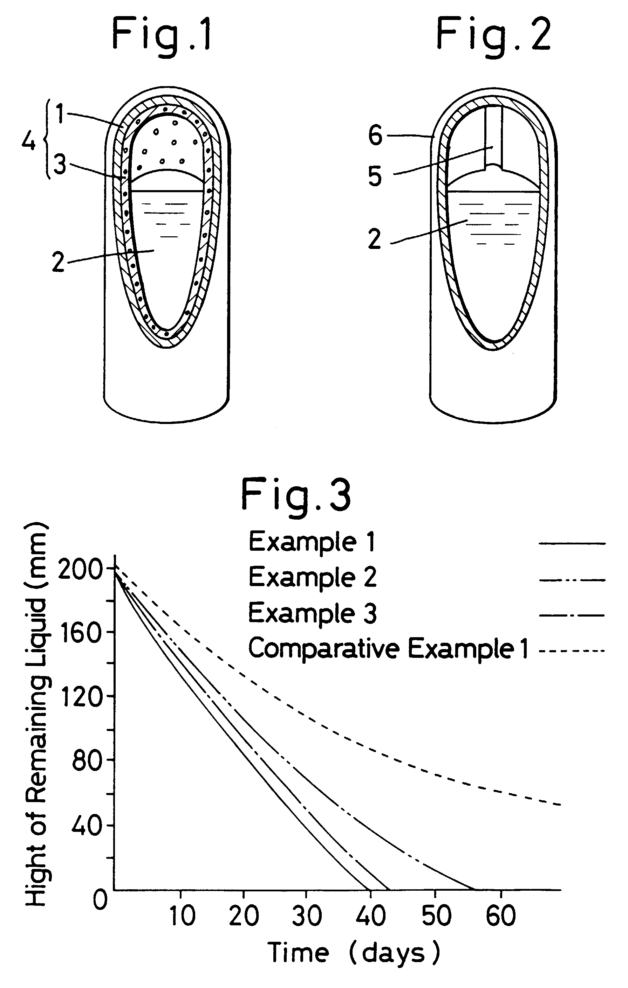

As will be seen from Fig. 1, the sustained release dispenser of the present invention comprises a plastic container 4, which has a liquid and volatile ingredient-permeable outer layer 1 and a liquid-absorbable inner layer 3 having materials identical to those of the outer layer 1, and a liquid and volatile ingredient 2 accommodated in an inner space being formed by the inner layer 3.

The inner layer 3 is a porous layer and the pores thereof are communicated to one another. The inner layer 3 can be prepared by extrusion molding in which the volatile ingredient 2 is incorporated into the layer when a molten plastic is extrusion-molded, or by foam extrusion-molding.

Another embodiment of the sustained release dispenser according to the present invention comprises, as shown in Fig. 2, a container 6 and a volatile ingredient 2 accommodated therein. A (plural) groove(s) is/are formed through from bottom end to top end on the inner wall of the container.

Materials used for preparing the plastic containers 4 and 6 may, for instance, be polyolefins such as polyethylenes and polypropylenes; ethylene-vinyl acetate copolymers; ethylene-acrylic acid ester copolymers; and ethylene-methacrylic acid ester copolymers. These raw materials may be used alone or in any combination. These plastic materials each is formed into a tube or bottle through extrusion molding or blow molding, which serves as an outer layer 1.

The volatile ingredient 2 may, for instance, be a sex pheromone. The sex pheromone is released through the container 4 or 6 to thus control any occurrence of agriculturally harmful insects.

The volatile ingredient 2 accommodated in the container 4 penetrates into the whole inner layer 3 through absorption in fine pores of the inner layer 3, then penetrates into the outer layer 1 through the inner layer and is finally released in the air from the surface of the outer layer 1. The ingredient 2 is absorbed in the whole inner layer 3 and hence penetrates into the whole outer layer 1 even if the liquid level of the ingredient 2 accommodated in the container 4 is lowered as the ingredient is released and therefore, the amount of the ingredient released from the container 4 is maintained at a constant level.

On the other hand, the ingredient 2 in the container 6 ascends along the groove 5 due to the capillary action, is absorbed in the container 6 and then released in the air from the outer surface of the container 6. Accordingly, the ingredient 2 ascends along the grooves 5, while being absorbed in the container 6 and released in the air even if the liquid level of the ingredient 2 accommodated in the container 6 is lowered as the release of the ingredient proceeds and therefore, the amount of the ingredient released from the container 6 is also maintained at a constant level.

Preferred embodiments of the present invention will hereunder be described in more detail, but the present invention is by no means limited to these specific embodiments.

Fig. 1 is a cross-sectional perspective view illustrating main parts of an embodiment of the sustained release dispenser according to the present invention. The sustained release dispenser is prepared by charging a pheromone as a volatile ingredient 2 in a hollow tube-like container 4 which comprises a plastic outer layer 1 and a plastic inner layer 3 and then sealing the container 4 by heat to thus enclose the ingredient 2 therein.

The inner layer 3 is a porous layer whose pores are communicated to one another. The porous layer may be prepared by the following methods. A first method comprises the step of injecting a volatile ingredient 2 compatible with a plastic material during extrusion molding the plastic into the porous layer. The temperature for the extrusion molding varies depending on the kinds of plastics, but in general ranges from about 130 to 300°C. For this reason, pheromones each having a boiling point of not less than 170°C can be used in the invention. The plastic is hardened while the plastic in the molten state comes in contact with the pheromone in this method and accordingly, the outer and inner layers 1 and 3 are produced from the same kind of plastics. The porous layer, i.e., the inner layer 3 has a thickness ranging from about 0.02 to 0.05 mm. This method permits the molding of the inner layer simultaneously with the charging of the pheromone and it is thus the most suitable method for mass production of the sustained release dispensers.

A second method is a molding method which makes use of the foam extrusion technique. The molding through foam extrusion comprises the step of simultaneously extruding both outer and inner layers in such a manner that a foaming agent-containing plastic is molded into an inner layer 3 and the plastic free of foaming agent is molded into an outer layer 1. The thickness of the porous layer serving as the inner layer 3 suitably ranges from 0.01 to 0.10 mm. The foaming agents usable herein may be, for instance, heat decomposable compounds such as azo compounds and volatile solvents such as hydrocarbons.

The size of the tube preferably has an inner diameter ranging from 0.5 to 4 mm, a thickness ranging from 0.1 to 2 mm and a length of not less than 50 mm because of easiness of production and easy handling. In this respect, if metal wires are fitted to the tube in parallel thereto, the tube can easily be bent and attached to plant bodies. The metal wire may be selected from those easily bendable and free of restoration through the elastic action, such as aluminum, copper and iron wires.

The tube is produced by extruding a molten plastic through a die, while supplying air to a mandrel positioned at the center of the die.

A bottle-like container is produced in the same way as the tube-like container. The surface area of the bottle-like container is smaller compared with that of the tube-like container, therefore the release rate of the ingredient 2 is correspondingly lowered. For this reason, the bottle-like container is suitable for accommodating an ingredient having relatively high volatility. The thickness of the bottle ranges from 0.1 to 2 mm like the foregoing tube while taking into consideration, for instance, stable release of the ingredient. The bottle-like container is in general molded by the blow molding technique.

The ingredient 2 is absorbed in the inner layer 3, then penetrates into the outer layer 1 and is finally released in the air. The ingredient 2 such as a pheromone absorbed in the inner layer 3 penetrates into the outer layer 1 by itself. Therefore, the wet area of the tube is not reduced and accordingly, the ingredient-release rate is not likewise lowered.

Fig. 2 is a cross-sectional perspective view illustrating main parts of another embodiment of the sustained release dispenser according to the present invention. The sustained release dispenser according to this embodiment is prepared by charging a volatile ingredient 2 in a hollow tube-like container 6 in which grooves 5 are formed on the inner wall thereof and then fusing the tube 6 to thus enclose the ingredient 2 therein.

The grooves 5 present on the inner wall of the container can be formed through the use of a mandrel having projections. The width and depth of each groove 5 may vary depending on the shape of the container, but both of them range from about 0.01 to 0.5 mm. The number of the grooves 5 to be formed is preferably as much as possible, but at least one groove may ensure a desired effect of the invention. If the container is subjected to melt-molding, the molded container is hardened while reducing the size of the grooves 5. Therefore, the projections must have a size 2 to 3 times that of the final groove 5.

The volatile ingredient 2 is, for instance, a pheromone and the plastic material for the container is polyethylene as in the case of the sustained release dispenser shown in Fig. 1. Most of the ingredient accommodated in the tube penetrates into the outer layer of the tube through the inner layer thereof and is then released in the air, but a part of the ingredient ascends along the grooves 5 due to the capillary action and is released in the air in the same manner. Accordingly, the ingredient-release rate is not lowered even if the amount of the ingredient remaining in the tube is reduced.

### Example 1

When a high density polyethylene (hereunder simply referred to as "polyethylene") was extrusion-molded, Z,Z/E-7,11-hexadecadienyl acetate (hereunder referred to as "HDDA") which is the sex pheromone of pink bollworm was injected to form a hollow tube whose inner layer was a porous layer. More specifically, the method was carried out as follows.

The polyethylene was extruded through a die at 200°C, while injecting HDDA maintained at 150°C through the mandrel positioned at the center of the die, then the extruded polyethylene was introduced into a water bath maintained at 70°C to thus continuously mold a HDDA-charged tube having an inner diameter of 0.80 mm and a thickness of 0.32 mm(including a porous layer having a thickness of 0.02 mm). The resulting tube was sealed at equal lengths of 200 mm and then cut into individual sustained release dispensers each containing 80 mg of HDDA enclosed therein.

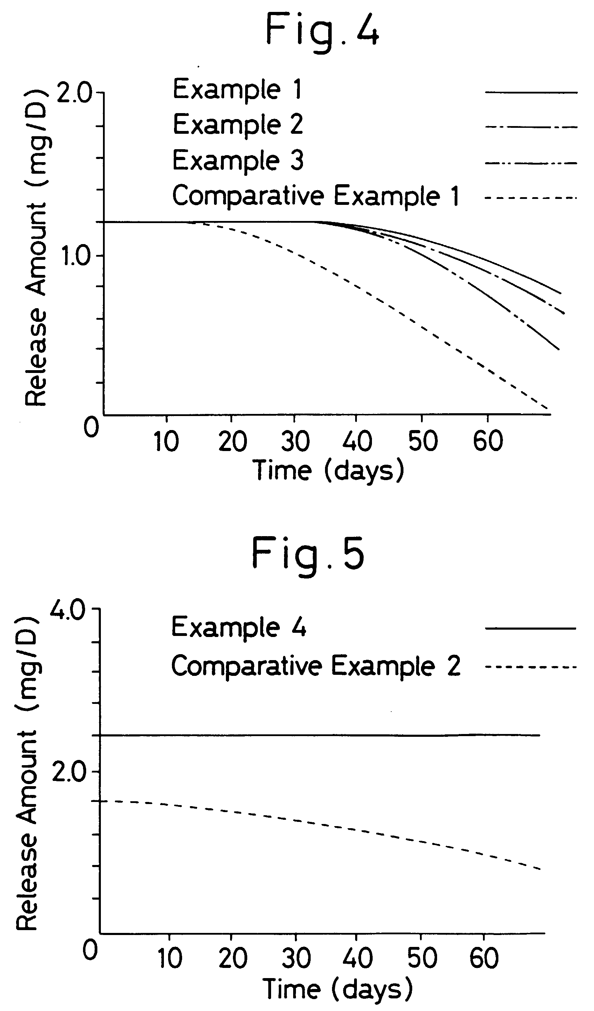

The dispenser was introduced into a thermostatic chamber maintained at 40 °C to determine the ingredient-release quality of the dispenser. Fig. 3 is a diagram showing a change, with time, of the height of the HDDA liquid remaining in the tube and Fig. 4 is a diagram showing a change, with time, of the amount of released HDDA. No liquid ingredient remained in the tube after 40 days as seen from Fig. 3, but the dispenser continued excellent release of HDDA even after 60 days as seen from Fig. 4. This was because a small amount of HDDA was still absorbed in the porous polyethylene layer. The rate of the remaining HDDA observed after 60 days was found to be 7%.

### Comparative Example 1

HDDA was introduced into a hollow tube whose inner wall was smooth and which had an inner diameter of 0.80 mm and a thickness of 0.30 mm. The conditions for the production of the hollow tube were identical to those used in Example 1 except that air of 20°C was substituted for the HDDA used in Example 1 during extrusion of the polyethylene. HDDA was charged in the hollow tube for 3 hours at a pressure of 8 kg/cm², and the tube was sealed and then cut at a length of 200 mm. Individual sustained releases dispenser were prepared each containing 80 mg of HDDA enclosed therein.

The ingredient-release quality of the dispenser was examined in the same manner used in Example 1. The results thus obtained are plotted on Figs. 3 and 4. There was observed marked reduction in the rate of ingredient-release with the lapse of time as seen from Fig. 4. At the same time, the reduction of the liquid remaining in the tube gradually became slower as seen from Fig. 3. The rate of the remaining HDDA observed after 60 days was found to be 25%.

### Example 2

In this Example, HDDA was charged in a hollow tube comprising porous inner layer formed through foam molding. The conditions for producing the hollow tube were identical to those used in Comparative Example 1 except that a polyethylene was extruded while azodicarbonamide as a foaming agent was added to a layer which would serve as the inner layer so that a porous layer formed had a thickness of 0.05 mm. The resulting tube was found to have an inner diameter of 0.80 mm and a thickness of 0.35 mm (including the porous layer having a thickness of 0.05 mm). The hollow tube was treated by the same method used in Comparative Example 1 to give sustained release dispensers each having a length of 200 mm and containing 80 mg of HDDA enclosed therein.

The ingredient-release quality of the dispenser was examined in the same manner used in Example 1. The results thus obtained are plotted on Figs. 3 and 4. As seen from Figs. 3 and 4, the ingredient-release quality of the dispenser was approximately identical to that of the dispenser produced in Example 1. The rate of the remaining HDDA observed after 60 days was found to be 10%.

### Example 3

A hollow tube was prepared by extrusion-molding a polyethylene in such a manner that grooves were formed on the inner wall of the resulting tube and then HDDA was charged therein. The conditions for the production of the hollow tube were identical to those used in Comparative Example 1 except that the extrusion molding was carried out using a mandrel having projections at 4 positions thereon. The hollow tube is thus provided with 4 grooves. Both of the width and depth of the groove are equal to 0.05 mm. The tube has an inner diameter of 0.80 mm and a thickness of 0.35 mm (including the depth (0.05 mm) of the inner groove). The hollow tube was treated by the same method used in Comparative Example 1 to give sustained release dispensers each having a length of 200 mm and containing 80 mg of HDDA enclosed therein.

The ingredient-release quality of the dispenser was examined in the same manner used in Example 1. The results thus obtained are plotted on Figs. 3 and 4. As seen from Figs. 3 and 4, the ingredient-release quality of the dispenser slightly approached that of the sustained release dispenser produced in Comparative Example 1. The rate of the remaining HDDA observed after 60 days was found to be 15%.

### Example 4

There was charged Z-8-dodecenyl acetate (hereunder referred to as "DDA"), i.e., the sex pheromone of Grapholita molesta Busck as a harmful insect for fruits in a polyethylene bottle whose inner layer was a porous layer produced by foam molding. The bottle was a cylindrical one having an outer diameter of 7.0 mm and a length of 15.0 mm and produced by simultaneously extrusion-molding a polyethylene for the inner layer containing azodicarbonamide as a foaming agent and a polyethylene for the outer layer . The resulting bottle had a thickness of 0.25 mm (including the porous layer having a thickness of 0.05 mm). There was charged 240 mg of DDA in the 0.50 mℓ volume bottle thus produced and then the opening for charging was sealed to give a sustained release dispenser.

The dispenser was introduced into a thermostatic chamber maintained at 40 °C to determine the ingredient-release quality of the dispenser. The results thus obtained are plotted on Fig. 5. Fig. 5 is a diagram showing a change, with time, of the amount of the released DDA. There was not observed any change in the amount of released DDA throughout the test. The rate of remaining DDA observed after 60 days was found to be 40%.

### Comparative Example 2

There was charged DDA in a bottle whose inner wall was smooth. The conditions for producing the bottle were identical to those used in Example 4 except that the extrusion molding was carried out using a polyethylene free of any foaming agent. After charging 240 mg of DDA into the cylindrical bottle having an outer diameter of 7.0 mm, a length of 15.0 mm, a thickness of 0.20 mm and a volume of 0.51 mℓ, the opening for charging was sealed to give a sustained release dispenser.

The ingredient-release quality of the dispenser was examined in the same manner used in Example 4. The results thus obtained are plotted on Fig. 5. As seen from Fig. 5, the amount of the released ingredient was small from the initiation of the test and reduced with time. The rate of the remaining DDA observed after 60 days was found to be 66%.

The sustained release dispenser according to the present invention allowed uniform release of a volatile liquid ingredient over a long time period even when the amount of the liquid ingredient remaining in the container was reduced and therefore, the ingredient could be used without waste. The sustained release dispenser ensured a uniform release rate over a long period of time and hence efficient mating disruption of harmful insects.

Herein disclosed is a sustained release dispenser capable of uniformly releasing a volatile liquid ingredient over a long time period even when the amount of the liquid ingredient remaining in a container is reduced. According to an embodiment, the sustained release dispenser comprises a plastic container 4 having a liquid and volatile ingredient-permeable outer layer 1 and a liquid-absorbable inner layer 3 having the same material as the outer layer 1 and a liquid and volatile ingredient 2 enclosed in an inner space formed by the inner layer 3. According to another embodiment, the sustained release dispenser comprises a plastic container 6 in which grooves 5 are formed on the inner wall and a volatile ingredient 2 accommodated in an inner space formed by the inner layer.

## Claims

1. A sustained release dispenser comprising a plastic hollow tube-like or bottle-like container (4), which comprises a liquid and volatile ingredient-permeable outer layer (1) as its outer surface and a liquid-absorbable inner layer (3) as its inner surface having the same material as the outer layer, and a liquid and volatile ingredient (2) accommodated in an inner space being formed by the inner layer.

2. The sustained release dispenser as set forth in claim 1 wherein the plastic is at least one compound selected from the group consisting of polyolefins, ethylene-vinyl acetate copolymers, ethylene-acrylic acid ester copolymers and ethylene-methacrylic acid ester copolymers.

3. The sustained release dispenser as set forth in claim 1 wherein the inner layer is a porous layer whose pores are communicated to one another.

4. The sustained release dispenser as set forth in claim 1 wherein the volatile ingredient is a sex pheromone.

5. A sustained release dispenser comprising a plastic container, of which grooves are formed through from a bottom to top on the inner wall, and a volatile ingredient accommodated in the container.

6. The sustained release dispenser as set forth in claim 5 wherein the plastic is at least one compound selected from the group consisting of polyolefins, ethylene-vinyl acetate copolymers, ethylene-acrylic acid ester copolymers and ethylene-methacrylic acid ester copolymers.

7. The sustained release dispenser as set forth in claim 5 wherein the volatile ingredient is a sex pheromone.

## Patentansprüche

1. Spender mit verzögerter Abgabe, der einen hohlen röhrenähnlichen oder flaschenähnlichen Behälter(4) aus Kunststoff, der als seine äußere Oberfläche eine äußere Schicht (1), die für flüssige und flüchtige Inhaltsstoffe permeabel ist, und eine Flüssigkeiten absorbierbare innere Schicht (3) als seine innere Oberfläche, die das gleiche Material wie die äußere Schicht aufweist, umfasst, und einen flüssigen und flüchtigen Inhaltsstoff (2), der in einem durch die innere Schicht gebildeten Innenraum untergebracht ist, umfasst.

2. Spender mit verzögerter Abgabe nach Anspruch 1, wobei der Kunststoff wenigstens eine Verbindung ist, die aus der aus Polyolefinen, Ethylen/Vinylacetat-Copolymeren, Ethylen/Acrylsäureester-Copolymeren und Ethylen/Methacrylsäureester-Copolymeren bestehenden Gruppe ausgewählt ist.

3. Spender mit verzögerter Abgabe nach Anspruch 1, wobei die innere Schicht eine poröse Schicht ist, dessen Poren miteinander verbunden sind.

4. Spender mit verzögerter Abgabe nach Anspruch 1, wobei der flüchtige Inhaltsstoff ein Sexpheromon ist.

5. Spender mit verzögerter Abgabe, der einen Behälter aus Kunststoff, bei dem Rillen durchgehend vom unteren Ende bis zum oberen Ende auf der Innenwand gebildet sind, und einen in dem Behälter untergebrachten flüchtigen Inhaltsstoff umfasst.

6. Spender mit verzögerter Abgabe nach Anspruch 5, wobei der Kunststoff wenigstens eine Verbindung ist, die aus der aus Polyolefinen, Ethylen/Vinylacetat-Copolymeren, Ethylen/Acrylsäureester-Copolymeren und Ethylen/ Methacrylsäureester-Copolymeren bestehenden Gruppe ausgewählt ist.

7. Spender mit verzögerter Abgabe nach Anspruch 5, wobei der flüchtige Inhaltsstoff ein Sexpheromon ist.

## Revendications

1. Distributeur à libération prolongée, comprenant un récipient (4) creux en matière plastique, en forme de tube ou en forme de bouteille, qui comprend une couche extérieure (1) perméable aux liquides et aux ingrédients volatils comme surface extérieure et une couche intérieure (3) apte à absorber les liquides comme surface intérieure, constituée de la même matière que la couche extérieure, et un ingrédient liquide et volatil (2) logé dans un espace intérieur formé par la couche intérieure.

2. Distributeur à libération prolongée répondant à la définition suivant la revendication 1, dans lequel la matière plastique consiste en au moins un composé choisi dans le groupe consistant en des polyoléfines, des copolymères éthylène-acétate de vinyle, des copolymères éthylène-ester d'acide acrylique et des copolymères éthylène-ester d'acide méthacrylique.

3. Distributeur à libération prolongée répondant à la définition suivant la revendication 1, dans lequel la couche intérieure est une couche poreuse dont les pores sont en communication les uns avec les autres.

4. Distributeur à libération prolongée répondant à la définition suivant la revendication 1, dans lequel l'ingrédient volatil est une phéromone sexuelle.

5. Distributeur à libération prolongée, comprenant un récipient en matière plastique, comportant des rainures qui sont formées d'une partie inférieure à la partie supérieure sur la paroi intérieure, et un ingrédient volatil logé dans le récipient.

6. Distributeur à libération prolongée répondant à la définition suivant la revendication 5, dans lequel la matière plastique consiste en au moins un composé choisi dans le groupe consistant en des polyoléfines, des copolymères éthylène-acétate de vinyle, des copolymères éthylène-ester d'acide acrylique et des copolymères éthylène-ester d'acide méthacrylique.

7. Distributeur à libération prolongée répondant à la définition suivant la revendication 5, dans lequel l'ingrédient volatil est une phéromone sexuelle.
